# EUROPÄISCHE PATENTSCHRIFT

(11) **EP 0 397 910 B1**
(45) Veröffentlichungstag und Bekanntmachung des Hinweises auf die Patenterteilung: **19.01.1994**
(21) Anmeldenummer: 89109074.8
(22) Anmeldetag: 19.05.1989
(51) Int. Cl.: A61B 17/39

(54) **Schaltungsanordnung zur Steuerung eines Elektro- Therapiegerätes, insbesondere HF-Chirurgiegerätes**
Control circuit for an electrotherapy apparatus, especially for a high-frequency surgical apparatus
Circuit de commande d'un appareil pour électrothérapie, en particulier d'un appareil chirurgical à H.F.

(43) Veröffentlichungstag der Anmeldung: 22.11.1990
(73) Patentinhaber: SIEMENS AKTIENGESELLSCHAFT, 80333 München (DE)
(72) Erfinder: Feucht, Peter, D-1000 Berlin 62 (DE)

(56) Entgegenhaltungen:
- DE-A- 2 429 021
- DE-A- 2 457 221
- DE-B- 2 457 900
- US-A- 4 378 801

## Beschreibung

Die Erfindung betrifft eine Schaltungsanordnung zur Steuerung eines Elektro-Therapiegerätes, insbesondere HF-Chirurgiegerätes, das wenigstens eine eine hohe Spannung führende Elektrode aufweist, an der wenigstens ein Schaltmittel angeordnet ist.

Bei bekannten derartigen Elektro-Therapiegeräten und HF-Chirurgiegeräten ist die Behandlungselektrode mit wenigstens einem Schaltmittel baulich beispielsweise an oder in einem Handgriff zusammengefaßt, um der behandelnden Person eine unmittelbare Einwirkungsmöglichkeit auf das Elektro-Therapiegerät zu geben, die sich im einfachsten Fall auf eine An-/Aus-Funktion beschränken kann. Aus Sicherheitsgründen, vor allem um die zu behandelnde Person vor der hohen Spannung der Elektrode zu schützen, ist diese im allgemeinen vom Potential, also auch von der Masse des Elektro-Therapiegerätes getrennt. Solche Elektroden sind regelmäßig harten Betriebsbedingungen ausgesetzt, beispielsweise im Freien bei Reanimation der Herztätigkeit durch Elektroschocks (Reizströme) oder auch in Operationsräumen beim Hantieren in oder an Operationswunden. In jedem Fall ist die Gefahr gegeben, daß Feuchtigkeit, im Freien beispielsweise in Form von Regen, im Operationsraum beispielsweise als Körperflüssigkeit, zu den Schaltmitteln an der Elektrode gelangen kann und diese durch Kriechströme bzw. Kurzschluß mit der hohen Spannung in ihrer sicheren Funktionsfähigkeit beeinträchtigt und/oder die zu behandelnde Person gefährdet.

Die Elektrode soll leicht zu handhaben sein. Regelmäßig weist sie mehr als ein Schaltmittel auf. Die harten Betriebsbedingungen, insbesondere durch Feuchtigkeitseinwirkungen, bei hohen Spannungen an der Elektrode erfordern einen großen Isolationsaufwand gegenüber den Schaltmitteln. Das führt zu einer unzumutbar großen sowie schweren und im Ergebnis schlecht zu handhabenden Elektrode. Dieser Nachteil ist vermeidbar, wenn die wirksame Isolation zwischen den Schaltmitteln und der hohen Spannung der Elektrode in das Elektro-Therapiegerät verlegt wird, wodurch aber eine Vielzahl von mit den Schaltmitteln leitend verbundenen Bauteilen (z.B. Relais sowie Bauteile zur Erzeugung der Betriebsspannung) isoliert werden muß.

Aus der DE-B-2 457 900 ist eine Schaltungsanordnung zur Steuerung eines HF-Chirurgiegerätes bekannt. Ein auf die Frequenz eines NF-Hilfssignales abgestimmtes Frequenzfilter enthält zur Potentialtrennung einen Transformator. Die Primärseite des Transformators bildet zusammen mit einem Schwingkreiskondensator den Emitterwiderstand einer daran angeschlossenen Transistorschaltstufe. Das Filter hat im Resonanzfall einen hohen Scheinwiderstand, der den Schalttransistor sperrt. Die Sekundärseite des Filters enthält ebenfalls einen Schwingkreiskondensator, der durch einen daran angeschlossenen Schalter kurzschließbar ist. Durch die dadurch entstehende Verstimmung der Resonanzfrequenz sinkt an der Primärseite des Filters der Scheinwiderstand und der daran angeschlossene Schalttransistor wird leitend, wodurch der HF-Generator geschaltet wird. Für jeden weiteren Schalter ist zur Potentialtrennung ein weiteres und zu isolierendes Frequenzfilter erforderlich.

Die DE-A-2 457 221 offenbart eine Schaltungsanordnung zur Stromartauswahl an einem HF-Chirurgiegerät durch einen zweipolig ausgebildeten Elektrodenhandgriff, in welchem zwei Tastschalter mit zwei Dioden antiparallel in Reihe geschaltet sind. Den Dioden wird aus einem Transformator eine NF-Nechselspannung zugeführt. Mit dem Sekundärkreis dieses Transformators sind nicht nur die Dioden nebst Tastschalter verbunden, sondern auch eine im Gehäuse des HF-Chirurgiegerätes gemeinsam mit einer Steuereinrichtung angeordnete Verblockung zur Sperrung der Hochfrequenz gegenüber der Steuereinrichtung. Die Steuereinrichtung ist aus zwei Relais mit je einem Haltekondensator und je einer dazu in Reihe geschalteten Diode gebildet. Die Gleichstrom durchlassende Verblockung ist mit Masse und mit dem HF-Generator verbunden und als Fünfpol ausgebildet. Dieser Fünfpol besteht aus zwei Drosseln und drei Kondensatoren. Daher ist ein relativ hoher Isolationsaufwand an diesen Bauteilen erforderlich.

Der Erfindung liegt die Aufgabe zugrunde, bei einer Schaltungsanordnung der eingangs beschriebenen Art eine Potentialtrennung zwischen Schaltmitteln und geschalteten Mitteln mit vermindertem Isolationsaufwand derart vorzunehmen, daß mehrere Schaltmittel an einer gemeinsamen Anordnung zur Potentialtrennung anschließbar werden.

Erfindungsgemäß wird diese Aufgabe gelöst, indem das Schaltmittel mit einem gleichrichtenden Bauteil in Reihe geschaltet und elektrisch leitend an eine Sekundärseite eines Wechselstrom übertragenden und Gleichstrom unterbrechenden Vierpoles angeschlossen ist, dessen Primärseite zugleich an eine Wechselstromquelle und an eine Auswerte- und Steuerschaltung angekoppelt ist, welche wenigstens eine durch Betätigung des Schaltmittels entstehende, stromrichtungsabhängige Rückwirkung über den Vierpol an der von dem Schaltmittel galvanisch getrennten Primärseite des Vierpoles auswertet und in Abhängigkeit vom Auswerteergebnis Funktionen im Elektro-Therapiegerät steuert.

Durch die erfindungsgemäße Ausbildung der Schaltungsanordnung ist lediglich ein isolierendes Bauteil, nämlich der Vierpol, zwischen Schaltmittel und geschaltetem Mittel nötig. Aufgrund der erfindungsgemäßen Schaltungstechnik kann dieser Vierpol den erschwerten Betriebsbedingungen (Feuchtigkeits- und/oder Spritzwassereinwirkung) entzogen und im Elektro-Therapiegerät, beispielsweise unmittelbar nach den Anschlußbuchsen am ElektroTherapiegerät, angeordnet werden, wodurch ein Vordringen der hohen Spannung auf andere Bauteile vermieden wird, die von den Schaltmitteln aus gesehen, hinter dem erfindungsgemäßen Vierpol liegen. Des weiteren sind in Verbindung mit gleichrichtenden Bauteilen mehrere Schaltmittel an nur einen Vierpol anschließbar, wodurch für die Übertragung von bis zu vier (Schalt-)Funktionen nur ein Bauteil im Elektro-Therapiegerät isoliert werden muß.

Die Schaltmittel können unterbrechbare Kontakte, Stecker (bei HF-Chirurgiegeräten beispielsweise steckbare Elektroden mit Bipolar/Monopolar-Umschaltung), Taster, Schalter od.dgl. sein. Der Vierpol kann als Transformator ausgebildet sein oder durch Kondensatoren, Filter u. dgl. realisiert werden; unter Anpassung an den jeweiligen Anwendungsfall. Von den durch sekundärseitige Laständerung entstehenden, richtungsabhängigen Rückwirkung über den Vierpol eignen sich zur Auswertung vor allem Spannungs- und/oder Stromänderungen an der Primärseite. Als gleichrichtende Bauteile eignen sich bevorzugt Halbleiterdioden.

Weitere Vorteile und Einzelheiten der Erfindung ergeben sich aus der nachfolgenden Beschreibung von Ausführungsbeispielen anhand der Zeichnungen und in Verbindung mit den Unteransprüchen.

Es zeigen:
Figur 1 den stark vereinfachten und schematisierten Signalflußweg einer erfindungsgemäßen Schaltungsanordnung,
Figur 2 ein stark vereinfachtes Schaltschema einer erfindungsgemäßen Schaltungsanordnung,
Figur 3 ein Prinzipschaltbild eines in der erfindungsgemäßen Schaltungsanordnung vorgesehenen Vierpoles,
Figur 4 ein Impulsdiagramm zu der Schaltungsanordnung nach Figur 2 und
Figur 5 eine schaubildliche Darstellung des konstruktiven Aufbaus eines als Ringkernimpuls-Transformator ausgebildeten Vierpoles gemäß Figur 2.

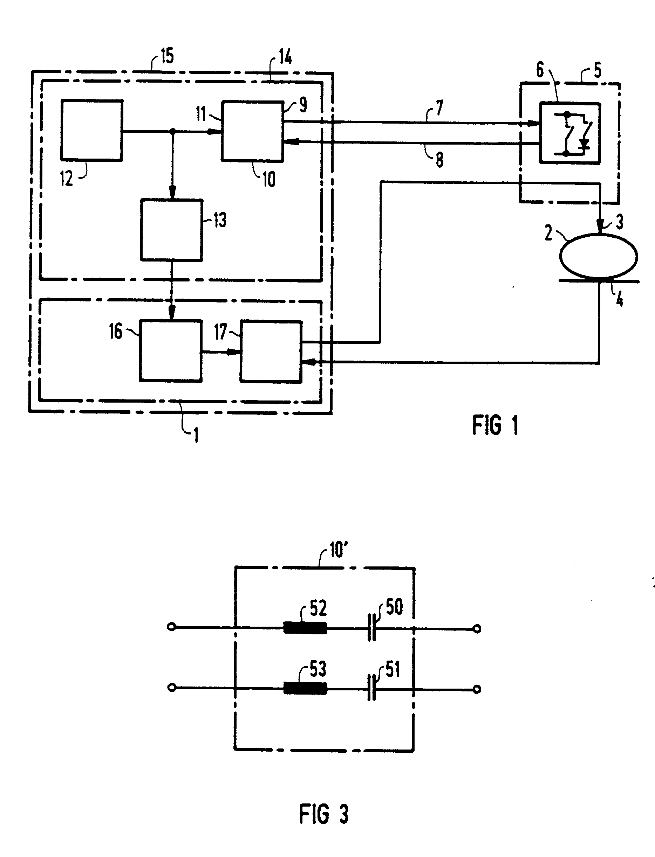

In Figur 1 wird mit einem Elektro-Therapiegerät 1 in bekannter Weise auf einen zu behandelnden Patienten 2 über Elektroden 3 und 4 eingewirkt, von denen wenigstens eine Elektrode 3 eine hohe Spannung führt, z.B. gegenüber der anderen Elektrode 4 und/oder gegenüber Massepotential. Die Elektrode 3 ist in einem Handgriff 5 gehalten, der auch Schaltmittel 6 enthält, von denen wenigstens eines mit einem gleichrichtenden Bauteil in Reihe geschaltet ist. Die Schaltmittel 6 sind galvanisch über eine Doppelleitung 7 und 8 mit der Sekundärseite 9 eines Vierpoles 10 verbunden, der Wechselstrom überträgt und Gleichstrom sperrt. Die Primärseite 11 des Vierpoles 10 ist an eine Wechselstromquelle 12 und an eine Auswerte- und Steuereinrichtung 13 angekoppelt, welche wenigstens eine durch Betätigung der Schaltmittel 6 entstehende Rückwirkung an der von den Schaltmitteln 6 galvanisch getrennten Primärseite 11 des Vierpoles 10 auswertet und in Abhängigkeit vom Auswerteergebnis Funktionen im Elektro-Therapiegerät 1 steuert, welches eine Energiequelle 16 und eine Ausgangsschaltung 17 enthält.

Die Wirkungsweise ist kurz folgende:
Wird in einem beispielhaft angenommenen ersten Fall ein Schaltmittel 6, z.B. ein Schalter, geschlossen, so liegt auf der Sekundärseite 9 des Vierpoles 10 ein Kurzschluß vor, der auf die Primärseite 11 zurückwirkt und dort unter anderem eine Stromänderung hervorruft, auf welche die Auswerte- und Steuerschaltung 13 ansprechen kann und eine dieser Stromänderung zugeordnete Steuerfunktion im Elektro-Therapiegerät 1 auslöst, z.B. die maximale Leistung einschaltet.

Wird in einem beispielhaft angenommenen zweiten Fall dagegen ein anderes Schaltmittel 6, das mit einer Diode in Reihe geschaltet ist, z.B. ein Taster, geschlossen, so liegt auf der Sekundärseite 9 des Vierpoles 10 für eine Stromflußrichtung annähernd ein Kurzschluß vor, der entsprechend auf die Primärseite 11 zurückwirkt und dort unter anderem eine Stromänderung in nur einer Stromflußrichtung hervorruft (der Effektivstrom ist geringer als im ersten Fall), worauf die Auswerte- und Steuereinrichtung 13 ansprechen kann (z.B. über Niederspannungs optokoppler) und eine dieser (richtungsabhängigen) Stromänderung, die von der nach dem ersten Fall verschieden ist, zugeordnete Steuerfunktion, die von der nach dem ersten Fall verschieden ist, im Elektro-Therapiegerät auslöst, z.B. eine verminderte Leistung einschaltet.

Des weiteren ist in Figur 1 angedeutet dargestellt, daß die zur erfindungsgemäßen Schaltungsanordnung gehörende Wechselstromquelle 12, die Auswerte- und Steuereinrichtung 13 sowie der Vierpol 10 als Baueinheit 14 räumlich dem Elektro-Therapiegerät 1 zugeordnet sind und ein gemeinsames Gehäuse 15 haben, das mit nicht dargestellten Anschlüssen für die Elektroden 3 und 4 sowie für die Schaltmittel 6 (Leitungen 7 und 8) ausgestattet ist.

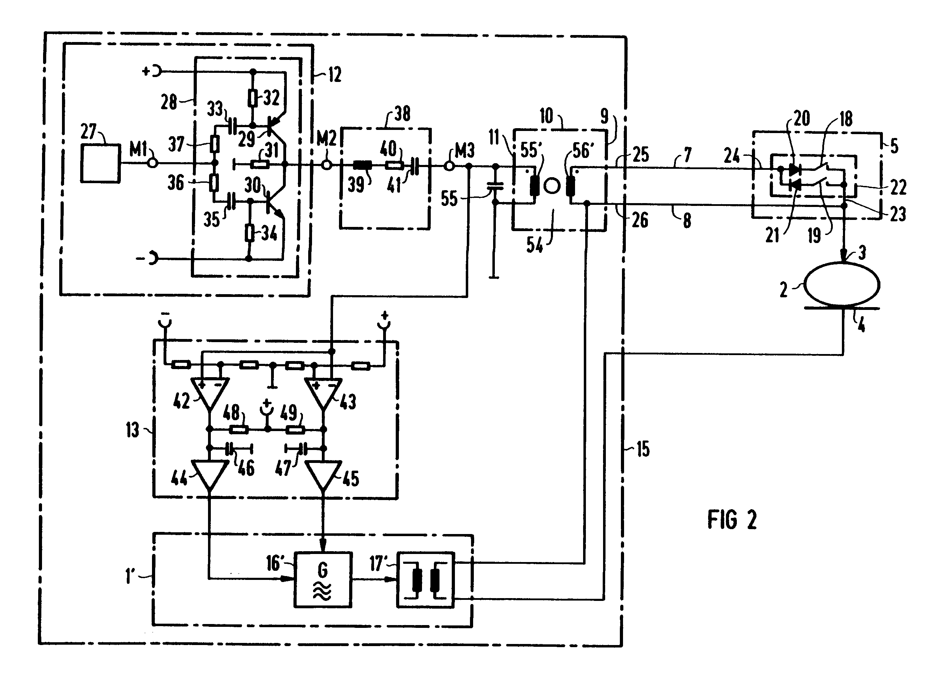

In einem schematisierten und vereinfachten Ausführungsbeispiel gemäß der Erfindung ist in Figur 2 die zu Figur 1 prinzipiell erläuterte Schaltungsanordnung ebenfalls enthalten und etwas detaillierter dargestellt. In einem gemeinsamen Gehäuse 15 sind ein HF-Chirurgiegerät 1′, die Auswerte- und Steuereinrichtung 13, die Wechselstromquelle 12 und der Vierpol 10 angeordnet. Das HF-Chirurgiegerät 1′ enthält unter anderem einen HF-Generator 16′, dem in bekannter Weise ein Transformator 17′ zur Potentialtrennung nachgeschaltet ist.

Als Schalter 18, 19 ausgebildete Schaltmittel im Handgriff 5 sind mit dem Vierpol 10 über die Doppelleitung 7, 8 verbunden. Über die Leitung 8 wird ferner die vom HF-Chirurgiegerät 1′ kommende Energie zur Elektrode 3 übertragen, wodurch der Handgriff 5 von einer Leitung entlastet und leichter zu handhaben ist.

Die Schalter 18 und 19 sind mit je einer Diode 20 und 21 in Reihe geschaltet und die beiden Reihenschaltungen antiparallel als Zweipol 22 zusammengeschaltet. Der eine Ausgang 23 des Zweipoles 22 ist mit der Elektrode 3 bzw. mit der zum HF-Chirurgiegerät führenden Leitung 8 galvanisch leitend verbunden. Der andere Ausgang 24 des Zweipoles 22 ist mit der Leitung 7 galvanisch verbunden, die zum Gehäuse 15 führt und über einen Anschluß 25 mit der Sekundärseite 9 des Vierpoles 10 verbunden ist. Ein zweiter Anschluß 26 der Sekundärseite 9 des Vierpoles ist mit der Leitung 8 galvanisch verbunden, die zur Elektro-de 3 führt. Damit führen die Dioden 20, 21 auch zu einer Einsparung von Übertragungsleitungen. Die Dioden 20 und 21 können auch im Gehäuse 15 sekundärseitig am Vierpol 10 angeordnet werden. Zur Verbindung mit den Schaltmitteln kann eine zusätzliche Leitung an bisherigen Elektroden-Handgriffen vorgesehen werden. Damit werden die bisherigen Elektroden-Handgriffe mit der erfindungsgemäßen Schaltungsanordnung kompatibel. Um die Dioden zu schützen bzw. um die Lastwirkung am Vierpol zu vermindern, kann ein nicht gezeichneter ohmscher Widerstand eingefügt werden.

Die Wechselstromquelle 12 ist im Ausführungsbeispiel nach Figur 2 als Impulsgeneratorschaltung mit einer sich anschließenden Ausgangsschaltung 28 ausgebildet, die eine Reihenschaltung aus Komplementärtransistoren 29 und 30 aufweist, deren Kollektoren über einen gemeinsamen Arbeitswiderstand 31 mit Masse verbunden sind. Ein Emitter ist mit einer positiven und der andere Emitter mit einer negativen Spannungsquelle verbunden. Durch je ein RC-Glied 32, 33 und 34, 35 an jeder Basis der Transistoren 29 und 30, z.B. Feldeffekttransistoren, wirken diese als monostabile Kippstufen, die durch positive und negative Impulsflanken aufweisende und der jeweiligen Basis über Widerstände 36, 37 von der Impulsgeneratorschaltung 27 zugeführte Impulse abwechselnd geschaltet werden. Dadurch kann der mittlere Leistungsverbrauch gegenüber einer Wechselstromquelle mit kontinuierlichem Wechselstrom reduziert werden, was auch zur Verminderung des Isolationsaufwandes am Vierpol beiträgt.

Der Ausgang der Wechselstromquelle 12 bzw. der Ausgangsschal tung 28 ist über Entkopplungsmittel 38, die aus einer Spule 39, einem Widerstand 40 und einem Kondensator 41 bestehen, an die Primärseite 11 des Vierpoles 10 angekoppelt. Durch die Ankopplung über die Entkopplungsmittel 38 können von den Schaltmitteln 6 ausgehende Rückwirkungen an der Primärseite 11 des Vierpoles 10 besser ausgewertet werden. Darüber hinaus können an den Ausgang der Wechselstromquelle 12 über weitere Entkopplungsmittel weitere Vierpole (mit sekundärseitigem anderem Potential) angeschlossen werden, wodurch von zusätzlichen Schaltmitteln an der gleichen oder an einer anderen Elektrode weitere Funktionen, z.B. Alarm- und/oder Überwachungsfunktionen, ausgelöst werden können. Es kann beispielsweise auch die Spule 39 als ein für mehrere Vierpole gemeinsames Entkopplungsmittel wirken, von der aus als separate Entkopplungsmittel Widerstände und/oder Kondensatoren mit weiteren Vierpolen gemäß der Erfindung verbunden sind.

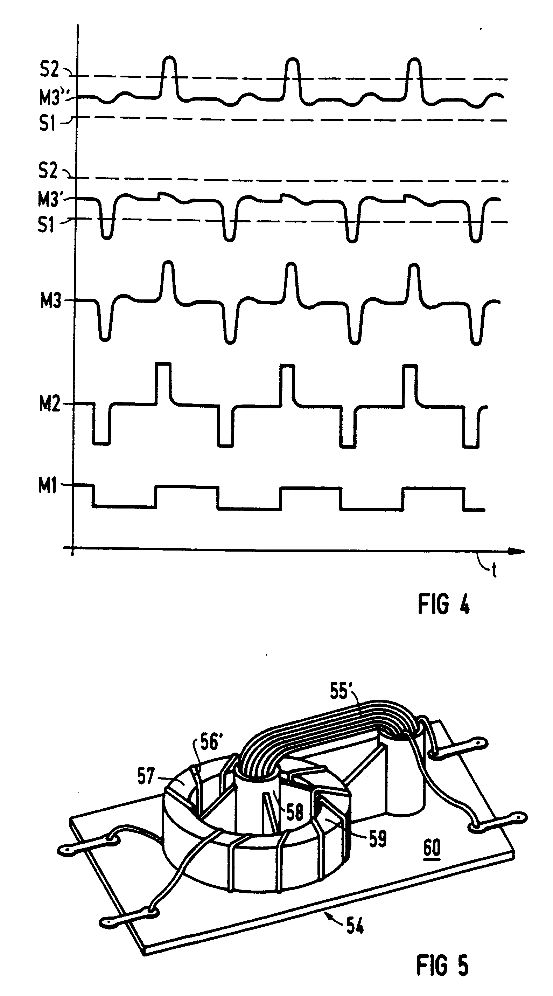

Die Auswerte-und Steuerschaltung 13 weist am Eingang zwei Komparatoren 42 und 43 auf, deren invertierender und nicht invertierender Eingang gemeinsam an die Primärseite 11 des Vierpoles 10 angekoppelt sind, wobei der eine Komparator 42 bei Überschreiten einer positiven Schwellenspannung S2 (siehe Figur 4) einen nachgeschalteten Logikeingang eines Gatterbausteines 44 gesperrt hält und der andere Komparator 43 bei Überschreiten einer negativen Schwellenspannung S1 einen Logikeingang eines anderen nachgeschalteten Gatterbausteines 45 gesperrt hält. Ladekondensatoren 46 und 47 werden über Widerstände 48 und 49 positiv und verzögert aufgeladen. Solange Impulse an den Komparatoreingängen anliegen, die betragsmäßig die vorgegebene Schwellenspannung S1 bzw. S2 überschreiten, sprechen die Komparatoren an und entladen die Kondensatoren 46 und 47, wodurch die Gatterbausteine 44 und 45 keine Funktion am HF-Generator auslösen. Wird dagegen einer der Schalter 18 oder 19 geschlossen, liegt an der Sekundärseite 9 des Vierpoles 10 für eine Stromflußrichtung nahezu ein Kurzschluß vor, dessen Rückwirkungen auf die Primärseite 11 die den Komparatoreingängen zugeführten Impulse verändern, wie es die beiden oberen Impulskurven in Figur 4 zeigen und was noch weiter unten detaillierter erläutert ist. Im Ergebnis wird der Kondensator 46 bzw. 47 nicht mehr entladen und der Gatterbaustein 44 bzw. 45 schaltet nach ausreichender Aufladung der Kondensatoren 46 bzw. 47 die ihm zugeordnete Funktion am HF-Generator 16′ bzw. HF-Chirurgiegerät 1′ ein. Werden beide Schalter 18 und 19 betätigt, werden im Ergebnis auch beide Funktionen am HF-Generator 16′ ausgelöst, die den Komparatoren 44 und 45 zugeordnet sind.

Um bei Ausfall des Wechselstromgenerators 12 ein Schalten von Funktionen durch die Gatterbausteine 44 und 45 zu verhindern, kann aus Sicherheitsgründen beispielsweise die positive Spannung den Widerständen 48 und 49 über eine nicht dargestellte Torschaltung zugeführt werden, die nur dann öffnet, wenn ein Wechselstromsignal am Ausgang der Wechselstromquelle 12 vorliegt.

Der Vierpol 10 kann elektrisch und/oder mechanisch verschieden ausgestaltet sein. Wenn lediglich hohe Gleichspannungen an der Elektrode auftreten, genügt ein Vierpol, der zwei Kondensatoren enthält. In Figur 3 ist zu den beiden Kondensatoren 50 und 51 jeweils eine Spule 52 und 53 in Reihe geschaltet. Dadurch kann dieser Vierpol 10′ auch in HF-Chirurgiegeräten eingesetzt werden, insbesondere, wenn die Serienresonanzkreise 50, 52 und 51, 53 auf die Frequenz (z.B. 4 kHz) der Wechselstromquelle 12 abgestimmt sind. Für die Hochfrequenz von z.B. 500 kHz wirkt der Vierpol als Sperrfilter.

Mit dem Vierpol 10 (siehe Figur 2) können insgesamt vier Informationen übertragen werden, nämlich "Aus" (Schalter 18 und 19 offen); nur Schalter 18 geschlossen; nur Schalter 19 geschlossen und Schalter 18 sowie 19 geschlossen. Dennoch braucht bei erfindungsgemäßen Schaltungsanordnung lediglich ein Bauteil, nämlich nur der Vierpol 10, ausreichend isoliert zu werden, abgesehen von den Anschlußbuchsen am Gehäuse 15.

In Figur 4 ist ein Impulsdiagram über der Zeitachse t dargestellt. Die untere Kurve zeigt Rechteckimpulse, wie sie am Meßpunkt M1 am Ausgang der Impulsgeneratorschaltung 27 im Ausführungsbeispiel gemäß Figur 2 gemessen werden können. An der Ausgangsschaltung 28 kann am Meßpunkt M2 eine Impulsfolge gemessen werden, die der zweiten Kurve von unten in Figur 4 entspricht. Die Impulsdauer ist klein gegenüber dem Abstand zum nächsten Impuls. Dadurch ist ausreichende Zeit für den Ablauf von Ladevorgängen innerhalb der Schaltungsanordnung gegeben. Die Impulsdauer der positiven und negativen Impulse ist gleich groß und die positive und negative Impulsspannung weist den gleichen Betrag auf. Dadurch lassen sich die Schwellspannungen S1 und S2 (siehe die beiden oberen Kurven in Figur 4) an den Komparatoren 42 und 43 einfach realisieren und die Entladevorgänge an den Kondensatoren 46 und 47 mit wenig Aufwand steuern. Die dritte Kurve von unten in Figur 4 zeigt das Impulssignal am Meßpunkt M3. Die Abrundungen und Überschwingungen entstehen durch die Entkopplungsmittel 38 und in Verbindung mit einem Kondensator 55, der vor allem restliche HF-Energie an der Primärseite eines Ringkernimpuls-Transformators 54 nach Masse kurzschließen soll.

Die beiden oberen Kurven M3′ und M3˝ sind ebenfalls am Meßpunkt M3 abgenommen, wobei im einen Fall der Schalter 19 und im anderen Fall der Schalter 18 geschlossen ist. Die strichlierten Linien stellen die jeweils einzustellende Schwellenspannung S1 und S2 an den Komparatoren 42 und 43 dar, um ein sicheres Schalten aufgrund der ankommenden Impulse, die bei anderer Ausgestaltung der Erfindung auch ein Wechselstromsignal sein können, zu ermöglichen.

Besonders einfach und kostengünstig ist ein Vierpol 10 als Ringkernimpuls-Transformator 54 ausgebildet. Dazu zeigen Figur 2 das elektrische Schema und Figur 5 eine mechanische Konstruktion. Die Primärwicklung 55′ und Sekundärwicklung 56′ des Ringkernimpuls-Transformators 54 können mit großem Abstand und geringer Kapazität gegeneinander auf dem Ringkern 57 angeordnet werden, wodurch auch bei Hochfrequenzspitzenspannungen, die bis an 10 kV heranreichen können, eine preisgünstige und sichere Potentialtrennung bei geringer Koppelkapazität der Wicklungen 55′ und 56′ realisierbar ist. Beispielsweise kann die Sekundärwicklung 56′ des Ringkernimpuls-Transformators 54 eng anliegend und über nahezu die gesamte Oberfläche des Ringkernes verteilt gewickelt werden und die andere Wicklung 55′ (Primärwicklung) in etwa bundförmig ähnlich einer Flachspule gewickelt werden, wobei ein Abschnitt der bundförmigen Wicklung durch das Zentrum 58 des Ringkernes verläuft und ein Abschnitt des Ringkernes wenigstens annähernd durch das Zentrum 59 der bundförmigen Wicklung. Dabei können der Ringkern 57 und die bundförmige Wicklung des Transformators 54 durch ein isolierendes und vorzugsweise einstückiges Kunststoff teil 60 in einer orthogonalen Lage zueinander gehalten werden, wodurch die sich dadurch ergebenden großen Abstände der Wicklungen 55′ und 56′ zueinander entscheidend zur Isolation auf einfachste Weise beitragen. Im übrigen kann das Kunststoffteil 60 so gearbeitet sein, daß sich lange Kriechstrecken für Leckströme zwischen den Wicklungen ergeben.

## Patentansprüche

1. Schaltungsanordnung zur Steuerung eines Elektro-Therapiegerätes (1, 1'), insbesondere HF-Chirurgiegerätes (1'), das wenigstens eine eine hohe Spannung führende Elektrode (3, 4) aufweist, an der wenigstens ein Schaltmittel (18, 19) angeordnet ist, das mit einem gleichrichtenden Bauteil (20, 21) in Reihe geschaltet ist und elektrisch leitend an eine Sekundärseite (9) eines Wechselstrom übertragenden und Gleichstrom unterbrechenden Vierpoles (10) angeschlossen ist, dessen Primärseite (11) zugleich an eine Wechselstromquelle (12) und an eine Auswerteund Steuereinrichtung (13) angekoppelt ist, welche wenigstens eine durch Betätigung des Schaltmittels (18, 19) entstehende, stromrichtungsabhängige Rückwirkung über den Vierpol (10) an der von dem Schaltmittel (18, 19) galvanisch getrennten Primärseite (11) des Vierpoles (10) auswertet und in Abhängigkeit vom Auswerteergebnis Funktionen im Elektro-Therapiegerät (1, 1') steuert.

2. Schaltungsanordnung nach Anspruch 1, bei der zu der Reihenschaltung aus dem Schaltmittel (18, 19) mit einem gleichrichtenden Bauteil (20, 21) ein weiteres Schaltmittel parallelgeschaltet und als ein die Sekundärseite (9) des Vierpoles (10) überbrückender Zweipol (6) geschaltet ist.

3. Schaltungsanordnung nach Anspruch 1, bei der zu dem Schaltmittel (18) mit einem in Reihe geschalteten gleichrichtenden Bauteil (20) eine weitere Reihenschaltung aus einem Schaltmittel (19) und einem gleichrichtenden Bauteil (21) antiparallel und als ein die Sekundärseite (9) des Vierpoles (10) überbrükkender Zweipol (22) geschaltet ist.

4. Schaltungsanordnung nach Anspruch 2 oder 3, bei der jedes gleichrichtende Bauteil (20, 21) räumlich einer Elektrode (3, 4) und die übrige Schaltungsanordnung räumlich dem Elektro-Therapiegerät (1, 1′) zugeordnet ist.

5. Schaltungsanordnung nach einem der Ansprüche 1 bis 4, bei der ein Anschluß des Schaltmittels (6) bzw. des Zweipoles (22) und ein Anschluß der Sekundärseite (9) des Vierpoles (10) mit einer Elektrode (3) bzw. mit dem zur Elektrode (3) führenden Leiter (8) galvanisch leitend verbunden ist.

6. Schaltungsanordnung nach einem der Ansprüche 1 bis 5, bei der die an die Primärseite (11) des Vierpoles (10) angekoppelte Auswerte- und Steuerschaltung (13) die rückwirkungsbedingten Stromaufnahmeänderungen erfaßt - vorzugsweise über wenigstens einen Niederspannungsoptokoppler - und auswertet.

7. Schaltungsanordnung nach einem der Ansprüche 1 bis 5, bei der die an die Primärseite (11) des Vierpoles angekoppelte Auswerte- und Steuerschaltung (13) die rückwirkungsbedingten Spannungsänderungen erfaßt und auswertet.

8. Schaltungsanordnung nach einem der Ansprüche 1 bis 7, bei der die Primärseite (11) des Vierpoles (10) über Entkopplungsmittel (Induktivität, ohmscher Widerstand od.dgl.) (38) an den Ausgang der Wechselstromquelle (12) angekoppelt ist, wobei an diesem Ausgang über weitere Entkopplungsmittel eine Mehrzahl von Vierpolen primärseitig angekoppelt sind und mit deren Sekundärseiten weitere Schaltmittel elektrisch leitend verbunden sind.

9. Schaltungsanordnung nach einem der Ansprüche 1 bis 8, bei der die Auswerte- und Steuerschaltung (13) am Eingang zwei Komparatoren (42, 43) aufweist, deren invertierender und nichtinvertierender Eingang gemeinsam an die Primärseite (11) des Vierpoles (10) angekoppelt sind, wobei
- der eine Komparator (42) bei Überschreiten einer positiven Schwellenspannung (S2) einen nachgeschalteten Logikeingang eines Gatterbausteines (44) gesperrt hält und
- der andere Komparator (43) bei Überschreiten einer negativen Schwellenspannung (S1) einen Logikeingang eines anderen nachgeschalteten Gatterbausteines (45) gesperrt hält.

10. Schaltungsanordnung nach einem der Ansprüche 1 bis 9, bei der die Auswerte- und Steuerschaltung (13) mit einer Torschaltung derart verknüpft ist, daß die Torschaltung mit der Wechselstromquelle (12) verbunden ist und nur bei Vorliegen eines Signals aus dieser Wechselstromquelle (12) öffnet und Steuersignale zum Steuern von Funktionen im Elektro-Therapiegerät (1, 1′) durchläßt.

11. Schaltungsanordnung nach einem der Ansprüche 1 bis 10, bei der der Vierpol (10) ein Ringkernimpuls-Transformator (54) ist und die Wechselstromquelle (12) eine Impulsgeneratorschaltung (27) ist, die Impulse mit wechselnder Polarität an die Primärwicklung (55′) des Transformators (54) abgibt, mit dessen Sekundärwicklung (56′) die Schaltmittel elektrisch leitend verbunden sind.

12. Schaltungsanordnung nach Anspruch 10, bei der die Impulsgeneratorschaltung (27) eine Ausgangsschaltung (28) aufweist, die aus zwei Komplementärtransistoren (29, 30) gebildet ist,
- deren Kollektoren leitend miteinander und über einen gemeinsamen Arbeitswiderstand (31), vorzugsweise ohmschen Widerstand, mit Masse verbunden sind,
- deren einer Emitter mit einer positiven und deren anderer Emitter mit einer negativen Spannungsquelle verbunden ist und
- jeder Transistor (29, 30) durch je ein RC-Glied (32, 33; 34, 35) an dessen Basis als monostabile Kippstufe wirkt,
wobei diese Kippstufen durch positive und negative Impulsflanken aufweisende und über die jeweilige Basis zugeführte Impulse abwechselnd geschaltet werden.

13. Schaltungsanordnung nach Anspruch 11 oder 12, bei der die Impulsdauer der an der Primärwicklung (55′) des Transformators (54) anliegenden Impulse klein ist gegenüber dem Abstand zum nächsten Impuls.

14. Schaltungsanordnung nach einem der Ansprüche 11 bis 13, bei der die Impulsdauer der an der Primärwicklung (55′) des Impulstransformators (54) anliegenden positiven Impulse wenigstens annähernd gleich groß ist wie die Impulsdauer der anliegenden negativen Impulse und bei der die positive und negative Impulsspannung (Impulshöhe) wenigstens annähernd den gleichen Betrag aufweist.

15. Schaltungsanordnung nach einem der Ansprüche 11 bis 14, bei der die Wicklungen (55′, 56′) des Ringkernimpuls-Transformators (54) mit großem Abstand und mit geringer Kapazität gegeneinander angeordnet sind.

16. Schaltungsanordnung nach Anspruch 15, bei der die eine Wicklung (56′) des Ringkernimpuls-Transformators (54) eng anliegend und über nahezu die gesamte Oberfläche des Ringkernes (57) verteilt gewickelt ist und die andere Wicklung (55′) in etwa bundförmig (ähnlich einer ringförmigen Flachspule) gewickelt ist, wobei ein Abschnitt der bundförmigen Wicklung (55′) durch das Zentrum (58) des Ringkernes (57) verläuft und ein Abschnitt des Ringkernes (57) wenigstens annähernd durch das Zentrum (59) der bundförmigen Wicklung (55′).

17. Schaltungsanordnung nach Anspruch 16, bei der der Ringkern (57) und die bundförmige Wicklung (55′) des Transformators (54) durch ein isolierendes und vorzugsweise einstückiges Kunststoffteil (60) in einer orthogonalen Lage zueinander gehalten werden.

18. Schaltungsanordnung nach Anspruch 16 oder 17, bei der die auf dem Ringkern (57) eng anliegende Wicklung (56′) als Sekundärwicklung und die bundförmige Wicklung (55′) als Primärwicklung des Ringkernimpuls-Transformators (54) geschaltet ist.

## Claims

1. Circuit arrangement for the control of an electrotherapy unit (1, 1'), in particular a high-frequency surgical unit (1'), which has at least one electrode (3, 4) conducting a high voltage, arranged at which electrode there is at least one switching means (18, 19) which is connected in series with a rectifying component (20, 21) and is connected in an electrically conductive manner to a secondary side (9) of a quadrupole (10) which transmits alternating current and interrupts direct current and the primary side (11) of which is coupled at the same time to an alternating current - source (12) and to an evaluating and control device (13) which evaluates at least one reaction, which develops as a result of actuation of the switching means (18, 19) and is dependent on current direction, by way of the quadrupole (10) on the primary side (11) of the quadrupole (10), which is electrically isolated from the switching means (18, 19), and controls functions in the electrotherapy unit (1, 1') as a function of the evaluation result.

2. Circuit arrangement according to claim 1, in which a further switching means is connected in parallel with the series circuit arrangement consisting of the switching means (18, 19) with a rectifying component (20, 21) and is connected as a two-port (6) bridging over the secondary side (9) of the quadrupole (10).

3. Circuit arrangement according to claim 1, in which a further series circuit arrangement consisting of a switching means (19) and a rectifying component (21) is connected in anti-parallel with the switching means (18) with a series-connected, rectifying component (20) and is connected as a two-port (22) bridging over the secondary side (9) of the quadrupole (10).

4. Circuit arrangement according to claim 2 or 3, in which each rectifying component (20, 21) is spatially coordinated with an electrode (3, 4) and the rest of the circuit arrangement is spatially coordinated with the electrotherapy unit (1, 1').

5. Circuit arrangement according to one of the claims 1 to 4, in which a terminal of the switching means (6) or of the two-port (22) respectively and a terminal of the secondary side (9) of the quadrupole (10) is connected in an electrically conductive manner to an electrode (3) and to the conductor (8) leading to the electrode (3) respectively.

6. Circuit arrangement according to one of the claims 1 to 5, in which the evaluating and control circuit arrangement (13) coupled to the primary side (11) of the quadrupole (10) detects the current consumption changes which are conditional on the reaction - preferably by way of at least one low-voltage optocoupler - and evaluates these.

7. Circuit arrangement according to one of the claims 1 to 5, in which the evaluating and control circuit arrangement (13) coupled to the primary side (11) of the quadrupole detects and evaluates the voltage changes which are conditional on the reaction.

8. Circuit arrangement according to one of the claims 1 to 7, in which the primary side (11) of the quadrupole (10) is coupled by way of decoupling means (inductor, ohmic resistor or the like) (38) to the output of the alternating current source (12), in which case a plurality of quadrupoles is coupled on the primary side to this output by way of further decoupling means, with further switching means being connected in an electrically conductive manner to the secondary sides of said quadrupoles.

9. Circuit arrangement according to one of the claims 1 to 8, in which the evaluating and control circuit arrangement (13) has at the input two comparators (42, 43), the inverting and non-inverting input of which are jointly coupled to the primary side (11) of the quadrupole (10), in which case
- the one comparator (42) keeps a subsequently connected logic input of a gate module (44) closed when a positive threshold voltage (S2) is exceeded, and
- the other comparator (43) keeps a logic input of another subsequently connected gate module (45) closed when a negative threshold voltage (S1) is exceeded.

10. Circuit arrangement according to one of the claims 1 to 9, in which the evaluating and control circuit arrangement (13) is linked with a gate circuit arrangement in such a way that the gate circuit arrangement is connected to the alternating current source (12) and only opens when a signal from this alternating current source (12) is present and allows control signals through to control functions in the electrotherapy unit (1, 1').

11. Circuit arrangement according to one of the claims 1 to 10, in which the quadrupole (10) is a toroidal-core pulse-transformer (54) and the alternating current source (12) is a pulse generator circuit arrangement (27) which emits pulses with alternating polarity to the primary winding (55') of the transformer (54), to the secondary winding (56') of which the switching means are connected in an electrically conductive manner.

12. Circuit arrangement according to claim 10, in which the pulse generator circuit arrangement (27) has an output circuit arrangement (28) which is formed of two complementary transistors (29, 30),
- the collectors of which are connected to each other in a conductive manner and are connected to earth by way of a common load resistor (31), preferably an ohmic resistor,
- the one emitter of which is connected to a positive voltage source and the other emitter of which is connected to a negative voltage source, and
- each transistor (29, 30) acts as a monostable flip-flop through a respective RC element (32, 33; 34, 35) at the base thereof,
in which case these flip-flops are switched alternately by means of pulses which have positive and negative pulse edges and which are supplied by way of the respective base.

13. Circuit arrangement according to claim 11 or 12, in which the pulse duration of the pulses applied at the primary winding (55') of the transformer (54) is small compared with the interval to the next pulse.

14. Circuit arrangement according to one of the claims 11 to 13, in which the pulse duration of the positive pulses applied at the primary winding (55') of the pulse transformer (54) is of at least substantially the same magnitude as the pulse duration of the negative pulses applied and in which the positive and negative pulse voltage (pulse height) is of at least approximately the same amount.

15. Circuit arrangement according to one of the claims 11 to 14, in which the windings (55', 56') of the toroidal-core pulse-transformer (54) are arranged with great interspacing and with low capacitance in respect of each other.

16. Circuit arrangement according to claim 15, in which the one winding (56') of the toroidal-core pulse-transformer (54) is wound so as to be tightly adjacent thereto and so as to be distributed over almost the whole surface of the toroidal core (57) and the other winding (55') is wound in a substantially bundle-shaped manner (similar to an annular flat coil), with one section of the bundle-shaped winding (55') extending through the centre (58) of the toroidal core (57) and one section of the toroidal core (57) extending at least substantially through the centre (59) of the bundle-shaped winding (55').

17. Circuit arrangement according to claim 16, in which the toroidal core (57) and the bundle-shaped winding (55') of the transformer (54) are held in an orthogonal position relative to each other by means of an insulating and preferably one-piece plastics portion (60).

18. Circuit arrangement according to claim 16 or 17, in which the winding (56'), which lies on the annular core (57) tightly adjacent thereto, is connected as a secondary winding and the bundle-shaped winding (55') is connected as a primary winding of the toroidal-core pulse-transformer (54).

## Revendications

1. Montage pour la commande d'un appareil électrothérapeutique (1,1'), notamment un appareil chirurgical à haute fréquence (1'), qui comporte au moins une électrode à haute tension (3,4), aux bornes de laquelle est monté au moins un dispositif de commutation (18,19), qui est branché en série avec un élément redresseur (20,21), et qui est branché, de manière électriquement conductrice, à une partie secondaire (9) d'un quadripôle (10) qui transmet le courant alternatif, qui interrompt le courant continu, et dont la partie primaire (11) est couplée en même temps à une source de courant alternatif (12) et à un dispositif d'exploitation et de commande (13), qui exploite, par l'intermédiaire du quadripôle (10), au niveau de la partie primaire (11), séparée galvaniquement du dispositif de commutation (18,19), une réaction dépendante de la direction du courant et créée par actionnement du dispositif de commutation (18,19), et commande, en fonction du résultat de cette exploitation, des fonctions dans l'appareil électrothérapeutique (1,1').

2. Montage suivant la revendication 1, dans lequel au circuit série, constitué du dispositif de commutation (18,19) et d'un élément redresseur (20,21), est branché en parallèle un autre dispositif de commutation, qui est branché en tant que bipôle (6) shuntant la partie secondaire (9) du quadripôle (10).

3. Montage suivant la revendication 1, dans lequel au dispositif de commutation (18) muni d'un élément de redressement (20) branché en série, est branché un autre circuit série constitué d'un dispositif de commutation (19) et d'un élément redresseur (21), de manière antiparallèle et en tant que bipôle (22) shuntant la partie secondaire (9) du quadripôle (10).

4. Montage suivant la revendication 1 ou 2, dans lequel chaque élément redresseur (20,21) est associé spatialement à une électrode (3,4), et le reste du montage est associé spatialement à l'appareil électrothérapeutique (1,1').

5. Montage suivant l'une des revendications 1 à 4, dans lequel une borne du dispositif de commutation (6) ou du bipôle (22) et une borne de la partie secondaire du quadripôle (10), sont reliées de manière galvaniquement conductrice, à une électrode (3) ou au conducteur (8) menant à l'électrode (3).

6. Montage suivant l'une des revendications 1 à 5, dans lequel le circuit d'exploitation et de commande (13), couplé à la partie primaire (11) du quadripôle (10), détecte - de préférence par l'intermédiaire d'au moins un optocoupleur de faible tension - et exploite les modifications de réception de courant obtenues par réaction.

7. Montage suivant l'une des revendications 1 à 5, dans lequel le circuit d'exploitation et de commande (13) couplé à la partie primaire (11) du quadripôle détecte et exploite les modifications de tension obtenues par réaction.

8. Montage suivant l'une des revendications 1 à 7, dans lequel la partie primaire (11) du quadripôle (10) est couplée, par l'intermédiaire de moyens de couplage (inductances, résistances ohmiques, ou semblable) (38), à la sortie de la source de courant alternatif (12), un grand nombre de quadripôles étant couplés, côté primaire, à cette sortie, par l'intermédiaire d'autres moyens de couplage, et d'autres moyens de commutation étant reliés de manière électriquement conductrice aux parties secondaires de ces quadripôles.

9. Montage suivant l'une des revendications 1 à 8, dans lequel le circuit d'exploitation et de commande (13) comporte, à l'entrée, deux comparateurs (42, 43) dont les entrées inverseuses et non inverseuses sont couplées ensemble à la partie primaire (11) du quadripôle (10), du type dans lequel
- le premier comparateur (42) maintient bloquée une entrée logique aval d'un élément de porte (44) lors du dépassement d'une tension de seuil positive (S2) et
- le deuxième comparateur (43) maintient bloquée une entrée logique d'un autre élément de porte (45) aval, lors du dépassement d'une tension de seuil négative (S1).

10. Montage suivant l'une des revendications 1 à 9, dans lequel le circuit d'exploitation et de commande (13) est relié à un circuit de porte de telle sorte que le circuit de porte est relié à la source de courant alternatif (12), n'est ouvert que dans le cas de la présence d'un signal issu de la source de courant alternatif (12) et n'est passant que dans le cas de la présence de signaux de commande pour la commande de fonctions dans l'appareil électrothérapeutique (1,1').

11. Montage suivant l'une des revendications 1 à 10, dans lequel le quadripôle (10) est un transformateur toroïdal à impulsions (54) et la source de courant alternatif (12) est un circuit générateur d'impulsions (27), qui fournit des impulsions ayant une polarité alternative aux bornes de l'enroulement primaire (55') du transformateur (54) avec l'enroulement secondaire duquel (56') sont reliés de manière électriquement conductrice les dispositifs de commutation.

12. Montage suivant la revendication 10, dans lequel le circuit générateur d'impulsions (27) comporte un circuit de sortie (28), qui est formé par deux transistors complémentaires (29,30),
- dont les collecteurs sont reliés de manière électriquement conductrice ensemble et à la masse par l'intermédiaire d'une résistance de travail commune (31), de préférence une résistance ohmique,
- dont un émetteur est relié à une source de tension positive et dont l'autre émetteur est relié à une source de tension négative, et
- chaque transistor (29,30) agit, par l'intermédiaire d'un circuit RC (32,33; 34,35) à leur base, en tant que bascule monostable,
- ces bascules étant commutées alternativement par des impulsions présentant des flancs d'impulsions positifs et négatifs et étant envoyées par l'intermédiaire de la base respective.

13. Montage suivant la revendication 11 ou 12, dans lequel la durée d'impulsions des impulsions appliquées à l'enroulement primaire (55') du transformateur (54) est inférieure à l'intervalle avec l'impulsion suivante.

14. Montage suivant l'une des revendications 11 à 13, dans lequel la durée d'impulsions des impulsions positives appliquées à l'enroulement primaire (55') du transformateur d'impulsions (54), est au moins approximativement aussi grande que la durée d'impulsions des impulsions négatives appliquées, et dans lequel les tensions d'impulsions (hauteur d'impulsions) positive et négative présentent au moins approximativement la même valeur.

15. Montage suivant l'une des revendications 11 à 14, dans lequel les enroulements (55', 56') du transformateur toroïdal à impulsions (54) sont placés les uns par rapport aux autres à grande distance et avec une petite capacité.

16. Montage suivant la revendication 15, dans lequel le premier enroulement (56') du transformateur toroïdal à impulsions (54) est enroulé en éventail en se resserrant et sur à peu près toute la surface du tore (57), et l'autre enroulement (55') est enroulé approximativement en forme de bobine (usuellement une bobine plate en forme d'anneau), une section de l'enroulement en forme de bobine (55') s'étendant à travers le centre (58) du tore (57) et une section du tore (57) s'étendant au moins approximativement à travers le centre de l'enroulement en forme de bobine (55').

17. Montage suivant la revendication 16, dans lequel le tore (57) et l'enroulement en forme de bobine (55') du transformateur (54) sont maintenus ensemble dans une position orthogonale, à l'aide d'une partie en plastique (60) isolante et, de préférence, d'un seul tenant.

18. Montage suivant la revendication 16 ou 17, dans lequel l'enroulement (56') se resserrant sur le tore (57) est branché en tant qu'enroulement secondaire du transformateur toroïdal à impulsion (54) et l'enroulement en forme de bobine (55') est branché en tant qu'enroulement primaire du transformateur toroïdal à impulsions (54).
